# EUROPEAN PATENT APPLICATION

(11) **EP 4 145 456 A1**
(43) Date of publication of application: **08.03.2023**
(21) Application number: 21194992.0
(22) Date of filing: 06.09.2021
(51) Int. Cl.: G16H 30/40, G16H 50/20

(54) **PREDICTION OF A CHANGE RELATED TO A MACULAR FLUID**

(71) Applicant: Bayer AG, 51373 Leverkusen (DE)
(72) Inventor: LENGA, Matthias, 24114 Kiel (DE); PROX, Jochen, 50825 Köln (DE); MOHAMMADI, Sadegh, 50968 Köln (DE); HOOGE, Jens, 13086 Berlin (DE); KRUPP, Alexander, 16542 Glienicke (DE); JOHSON, Kristian, 02142 Cambridge, MA (US)
(74) Representative: BIP Patents

(57) **Abstract**

Systems, methods, and computer programs disclosed herein relate to training and using a machine learning model to predict changes related to macular fluid in an eye of a patient suffering from an eye disease, in particular suffering from wet AMD.

## Description

### FIELD

Systems, methods, and computer programs disclosed herein relate to training and using a machine learning model to predict changes related to macular fluid in an eye of a patient suffering from an eye disease, in particular suffering from wet AMD.

### BACKGROUND

Age related macular degeneration (AMD) is a medical condition that usually affects older adults and results in a loss of vision in the center of the visual field (the macula) because of damage to the retina. It occurs in "dry" and "wet" forms. In the dry form, cellular debris called drusen accumulates between the retina and the choroid, and the retina can become detached. The wet form (wet AMD, also referred to as neovascular age-related macular degeneration) is characterized by new blood vessel formation in the macula of the eye. Neovascular leakage allows fluid to accumulate in the macula manifesting as intraretinal fluid or fluid pooling underneath the retina as subretinal fluid. Liquid in these compartments affects visual acuity.

The proliferation of abnormal blood vessels in the retina can be stimulated by vascular endothelial growth factor (VEGF). Inhibiting the angiogenic-promoting properties of VEGF appears to be an effective strategy for treating angiogenic eye disorders. Antiangiogenics or anti-VEGF agents can cause regression of the abnormal blood vessels and improve vision. Several anti-VEGF drugs have been approved for use in the eye: Aflibercept (Eylea^{®}), Bevacizumab (Avastin^{®}), Ranibizumab (Lucentis^{®}), Brolucizumab (Beovu^{®}), Conbercept (Langmu^{®}), Pegaptanib (Macugen^{®}).

Macular fluid is considered a useful biomarker in wet AMD management (see e.g. L. Kodjikian et al.: Fluid as a critical biomarker in neovascular age-related macular degeneration management: literature review and consensus recommendations, Eye, 2021, 35:2119-2135). Diagnosis, prognosis and/or treatment methods are often based on analysis of the amount and/or distribution of macular fluid.

Since the widespread introduction of optical coherence tomography (OCT) for the visualisation of the back of the eye in patients with eye diseases such as wet AMD, the evaluation of lesion morphology using OCT has become a key part of the clinical decision-making pathway. Markers for disease activity based on OCT, including intraretinal and subretinal as well as subretinal pigment epithelium fluid, are crucial for guiding management and treatment frequency of patients suffering from wet AMD.

Artificial intelligence (AI) has become available to a larger community of researchers worldwide owing to improvements in computer hardware and software and represents a breakthrough in big data management. Machine learning models are used for automated image analysis and allow evaluation of large amounts of data.

T. Schlegl *et al.* describe the use of a machine learning model for automatic detection and quantification of macular fluid in OCT images (Fully Automated Detection and Quantification of Macular Fluid in OCT Using Deep Learning, Ophthalmology, Volume 125, Issue 4, 2018, pages 549-558).

WO2021026039A1 discloses the use of a machine learning model for predicting current or visual acuity of a subject on the basis of an image of at least part of an eye of the subject.

WO2021113674A1 discloses the use of a machine learning model for predicting, on the basis of OCT images, frequency and/or interval at which a treatment (e.g. anti-vascular endothelial growth factor therapy) is to be administered so as to prevent leakage of vasculature in the eye of a patient.

WO2019210079A1 discloses the use of a machine learning model for predicting the development and progression of retinal disease on the basis of OCT data.

WO2019075410A1 discloses the use of a machine learning model for diagnosing ophthalmic diseases on the basis of ophthalmic images (WO2019075410A1).

D. B. Russakoff *et al.* disclose the use of a machine learning model for predicting the likelihood of converting from early/intermediate to advanced wet AMD on the basis of OCT images (Deep Learning for Prediction of AMD Progression: A Pilot Study, Investigative Ophthalmology & Visual Science February 2019, Vol.60, pages 712-722).

A disadvantage of the approaches described above is that the predicted features are not immediately apparent from OCT data. For a doctor it is often incomprehensible which information caused a model to make this or that prediction. As a result, a doctor may be suspicious of such a prediction.

Usually, a doctor does not want to leave a diagnosis, prognosis and/or the selection of a treatment method to a model, but rather to keep control.

Furthermore, the more unclear the relationship between the prediction result and the data on which the prediction is based, the less accurate the prediction result may be.

### BRIEF SUMMARY

This problem is addressed by the present disclosure.

The approach of the present disclosure is to predict characteristics of a patient that a doctor can understand immediately and that enable him to better diagnose a disease, prognose the development of a disease and/or select therapeutic approaches for treating a disease.

In a first aspect, the present disclosure provides a computer-implemented method for predicting a change of a macular fluid in an eye of a patient, the method comprising the steps:
- providing a trained machine learning model,
- receiving patient data, the patient data comprising information about a macular fluid in an eye of a patient,
- inputting the patient data into the trained machine learning model,
- receiving from the trained machine learning model information about a change related to the macular fluid in the eye of the patient,
- outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.

In another aspect, the present disclosure provides a computer system comprising:
a processor; and
a memory storing an application program configured to perform, when executed by the processor, an operation, the operation comprising:
   - receiving patient data, the patient data comprising information about a macular fluid in an eye of a patient,
   - inputting the patient data into the trained machine learning model,
   - receiving from the trained machine learning model information about a change related to the macular fluid in the eye of the patient,
   - outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.

In another aspect, the present disclosure provides a non-transitory computer readable medium having stored thereon software instructions that, when executed by a processor of a computer system, cause the computer system to execute the following steps:
- receiving patient data, the patient data comprising information about a macular fluid in an eye of a patient,
- inputting the patient data into the trained machine learning model,
- receiving from the trained machine learning model information about a change related to the macular fluid in the eye of the patient,
- outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.

### DETAILED DESCRIPTION

The invention will be more particularly elucidated below without distinguishing between the aspects of the invention (method, computer system, computer-readable storage medium). On the contrary, the following elucidations are intended to apply analogously to all the aspects of the invention, irrespective of in which context (method, computer system, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will now be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for predicting a change related to macular fluid in the eye of a patient.

The patient can be a human being suffering from an eye disease. In a preferred embodiment, the patient is suffering from wet AMD.

It is possible that only one eye of the patient is affected by a disease or that both eyes are affected by a disease. In case of both eyes being affected by a disease, the severity of the disease and/or the progress of the disease and/or the progress of a therapy for curing the disease or relieving symptoms of the disease can be the same or may be different.

The term "macular fluid" as it is used herein, means intraretinal fluid, subretinal fluid and/or subretinal pigment epithelium fluid.

"Intraretinal fluid" either can occur diffusely (diffuse intraretinal fluid) and can cause increased retinal thickness and reduced retinal reflectivity or be localized or non-reflective cysts (cystic edema, focal intraretinal fluid).

"Subretinal fluid" corresponds to the accumulation of a clear or lipid-rich exudate (serous fluid) in the subretinal space, i.e., between the neurosensory retina and the underlying retinal pigment epithelium, in the absence of retinal breaks, tears, or traction.

"Subretinal pigment epithelium fluid" corresponds to the accumulation of fluid (blood, serous exudate, drusen) between retinal pigment epithelium and Bruch membrane and it can cause retinal pigment epithelial detachment.

In an embodiment of the present disclosure, a change related to a specific type of macular fluid is predicted. In a preferred embodiment of the present disclosure, changes related to different types of macular fluid are predicted. In a preferred embodiment of the present invention, the computer system is configured to identify different types of macular fluid in an eye of a patient and to predict changes related to the different types of macular fluid.

The term "change" as it is used herein can be any change in nature, texture, composition, shape, quantity, and/or spatial distribution of (one or more) macular fluid(s) over time.

A change related to a macular fluid can be e.g. a change of a volume of a macular fluid compartment.

A change related to a macular fluid can be e.g. a change of a size and/or shape of a macular fluid compartment.

A change related to a macular fluid can be e.g. a change of a number of macular fluid compartments in a region of interest.

A change related to a macular fluid can be e.g. a change of an appearance of macular fluid compartments in a region of interest.

Any change can be caused by a (specific) treatment of the diseased eye or it can be caused by non-treatment (omission of treatment).

The present invention enables an ophthalmologist to study the influence of a (particular) therapy on one or more macular fluids in an eye of a patient. The ophthalmologist can generate a prediction that tells him/her how one or more macular fluids in a patient's eye will develop over time if the ophthalmologist takes this or that measure.

In a preferred embodiment, the predicted change is any change of a macular fluid in the eye of the patient caused by a treatment of the eye with a certain dose of a VEGF antagonist and/or caused by non-treatment.

As used herein, the expression "VEGF antagonist" means any molecule that blocks, reduces, neutralizes, inhibits, abrogates, or interferes with the normal biological activity of one or more member of the VEGF family including its binding to one or more VEGF receptors (VEGFR1 and VEGFR2). VEGF antagonists include for example molecules which interfere with the interaction between a VEGF and its natural VEGF receptor, e.g. molecules which bind to a VEGF or a VEGF receptor and prevent or otherwise hinder the interaction between the VEGF and its VEGF receptor.

VEGF antagonists (or anti-VEGF) include
( i) antibody VEGF antagonists such as but not limited to
   - anti-VEGF antibodies such as bevacizumab (Avastin^{®}; WO9845331) and antigen-binding fragments thereof such as ranibizumab (Lucentis^{®} WO9845331), brolucizumab (Beovu^{®}, EP2307454),
   - anti-VEGFRl or anti-VEGFR2 antibodies or and antigen-binding fragments thereof
( ii) non-antibody VEGF antagonists such as but not limited to
   - small molecule inhibitors of the VEGFR tyrosine kinases (e.g. sunitinib),
   - RNA aptamers specific to VEGF,
   - Antibody-Mimetika against VEGF or VEGF receptors (e.g. Affibody^{®}-molecules (e.g. DARPin^{®} MP0112 (WO2010/060748)), Affiline, Affitine, Anticaline, Avimere), and
   - VEGF receptor-based chimeric molecules also known as VEGF fusion proteins or VEGF-Traps such as Aflibercept (Eylea^{®}; WO2000/75319) or conbercept (Langmu^{®}, WO2005/121176).

VEGF receptor-based chimeric molecules include chimeric polypeptides which comprise two or more immunoglobulin (Ig)-like domains of a VEGF receptor such as VEGFR1 (also referred to as Flt1) and/or VEGFR2 (also referred to as Flk1 or KDR), and may also contain a multimerizing domain, e.g. an Fc domain which facilitates the multimerization, e.g. dimerization of two or more chimeric polypeptides. Exemplary VEGF receptor-based chimeric molecules are Aflibercept and conbercept.

In a preferred embodiment of the present invention, the VEGF antagonist is Aflibercept.

A change related to a macular fluid is predicted by means of a machine learning model.

Such a "machine learning model", as used herein, may be understood as a computer implemented data processing architecture. The machine learning model can receive input data and provide output data based on that input data and the machine learning model, in particular parameters of the machine learning model. The machine learning model can learn a relation between input and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning mode" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training to evaluate the machine learning model. For example, a loss function can include a metric of comparison of the output and the target. The loss function may be chosen in such a way that it rewards a wanted relation between output and target and/or penalizes an unwanted relation between an output and a target. Such a relation can be e.g. a similarity, or a dissimilarity, or another relation.

A loss function can be used to calculate a loss value for a given pair of output and target. The aim of the training process can be to modify (adjust) parameters of the machine learning model in order to reduce the loss value to a (defined) minimum.

A loss function may for example quantify the deviation between the output of the machine learning model for a given input and the target. If, for example, the output and the target are numbers, the loss function can be the difference between these numbers, or alternatively the absolute value of the difference. In this case, a high absolute value of the loss function can mean that a parameter of the model needs to undergo a strong change.

In the case of a scalar output, a loss function may be a difference metric such as an absolute value of a difference, a squared difference.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

In the case of higher dimensional outputs, such as two-dimensional, three-dimensional or higher-dimensional outputs, for example an element-wise difference metric may be used. Alternatively or additionally, the output data may be transformed, for example to a one-dimensional vector, before computing a loss function.

In case of the present disclosure, the training data comprise, for each subject of a multitude of subjects, first subject data and second subject data.

A "subject" can be a human being suffering from an eye disease. In a preferred embodiment, a subject is a patient suffering from wet AMD.

The term "subject" is used in this description in connection with the training of the machine learning model, whereas the term "patient" is used in this description in connection with the use of the trained machine learning model for making predictions. In other words: a subject is a patient from whom subject data are used to train a machine learning model. Once the machine learning model is trained, it can be used for making predictions for a (new) patient. So, the distinction between "subject" and "patient" is made herein only for the sake of clarity. Apart from this formal distinction between a "subject" and a patient", everything that is written about a "subject" applies analogously to a "patient" and *vice versa.*

The term "multitude" as it is used herein means an integer greater than 1, usually greater than 10, preferably greater than 100.

First subject data comprise information about a macular fluid in an eye of the subject at a first point in time. Second subject data comprise information about the macular fluid in the eye of the subject at a second point in time. Preferably, the second point in time is after (later than) the first point in time.

The time period between the first point in time and the second point in time can e.g. be a number of days, such as 1 day or 2 days or 3 days or 4 days or 5 days of 6 days or 7 days or 8 days or 9 days or 10 days or 11 days or 12 days, or a number of weeks, such as 1 week or 2 weeks or 3 weeks or 4 weeks or 5 weeks or 6 weeks, or a number of months, such as 1 month or 2 months or 3 months or 4 months or 5 months or 6 months or 7 months or 8 months or 9 months or 10 months or 11 months or 12 months, or a number of years, such as 1 year or 2 years or 3 years, or a combination thereof (such as for example a time period of 2 months + 1 week + 5 days).

The first time point (=first point in time) and/or the second time point (=second point in time) can be related to an event, such as a visit of the subject to an ophthalmologist, and/or a medical examination of the subject and/or a medical treatment of the subject.

The machine learning model is trained to predict second subject data from first subject data. In other words, first subject data are used as input data and second subject data are used as target. The machine learning model is trained to generate, on the basis of first subject data, output data which come as close as possible to the subject data (the target).

The training of the machine learning model usually comprises the following steps: for each subject of the multitude of subjects:
- inputting first subject data into the machine learning model,
- receiving from the machine learning model output data,
- computing a loss value using a loss function, the loss value quantifying the deviation(s) of the output data from the second subject data,
- modifying parameters of the machine learning model in a way that minimizes the loss value.

In a preferred embodiment, subject data characterizing macular fluid in an eye of a subject at a plurality of time points is used for training the machine learning model.

The term "plurality" of time points means a number N of time points, wherein N is an integer equal to or greater than 2, for example, N = 2, 3, 4, 5, 6, 7, 8, 9, 10, 11, 12, 13, 14, 15, 16, 17, 18, 19, 20 or any other number.

For example, the subject data used for training may comprise first subject data characterizing macular fluid in an eye of a subject at a first point in time, second subject data characterizing macular fluid in the eye of the subject at a second point in time, and third subject data characterizing macular fluid in the eye of the subject at a third point in time, wherein the third point in time is after (later than) the second point in time, and the second point in time is after (later than) the first point in time.

The subject data may further comprise fourth subject data characterizing macular fluid in the eye of the subject at a fourth point in time, wherein the fourth point in time is after (later than) the third point in time. The subject data may further comprise fifth subject data characterizing macular fluid in the eye of the subject at a fifth point in time, wherein the fifth point in time is after (later than) the fourth point in time. And so on.

The time period between different time points can be the same or different.

In case of subject data comprising first subject data, second subject data and third subject data, first subject data and second subject data may be used as input data, from which output data is generated by the machine learning model which comes as close as possible to third subject data. In this case, third subject data is used as target.

In case of subject data comprising first subject data, second subject data, third subject data, and fourth subject data, first subject data, second subject data and third subject data may be used as input data, from which output data is generated by the machine learning model which comes as close as possible to fourth subject data. In this case, fourth subject data is used as target.

In other words, the model can be trained to learn how a macular fluid develops over time, and it can be trained to extrapolate the status of the macular fluid at a later point in time.

Subject data characterizes a macular fluid in an eye of a subject at a certain point in time (e.g. at a first point in time or at a second point in time or at a third point in time, and so forth). In a preferred embodiment, information about the point in time at which subject data characterizes the macular fluid in the eye of the subject (time stamp) is used as additional training data for training the machine learning model. For example, the date and optionally the time on which / at which first subject data and second subject data (and, if available, third, fourth, ... subject data) were generated is inputted as additional input data into the machine learning model.

Additionally or alternatively, information about the time period between different sets of subject data can be used as additional training data for training the machine learning model, for example the time period between the first point in time and the second point in time and the time period between the second point in time and the third point in time (if existing).

It also possible that subject data were collected at defined time intervals, e.g. every two weeks or every month or at other (regular) time intervals. In case the time period between different points in time is a constant (the same for all sets of subject data), information about the different time points or the time periods between the time points may not be used as additional input data.

If the date/time on/at which subject data were generated is related to a certain event, information about the event can be used as additional input data. If, for example, the subject data were generated on a day on which the subject received a medical treatment (e.g. shortly before or after the subject data were generated), information about the medical treatment (e.g. medicament, dosage, number of treatments received before) can be used as additional input data. If no treatment was carried out, the information that no treatment was carried out, can be used as additional input data. If, for example, subject data were generated on a day on which a doctor made a diagnosis, information about the diagnosis (e.g. diagnosed disease, severity of the disease, cause(s) of the disease, side effects of the disease, recommended method of treatment, previous treatment) can be used as additional input data.

In a preferred embodiment of the present invention, the first subject data and the second subject data (and further subject data if present) comprise one or more image(s) of an eye, or a part thereof, of the subject.

The term "image" as used herein means a data structure that represents a spatial distribution of a physical signal. The spatial distribution may be of any dimension, for example 1D, 2D, 3D, 4D or any higher dimension. The spatial distribution may be of any shape, for example forming a grid and thereby defining pixels, the grid being possibly irregular or regular.

The image is a representation of the subject's eye or a part thereof. In such an image, macular fluid can be identified. Preferably, different types of macular fluids can be identified.

Examples of an image for identification of macular fluid include one or more optical coherence tomography (OCR) image(s), fluorescein angiography (FA) image(s), infra-red reflectance (IR) image(s), infra-red autofluorescence (IR-AF) image(s), near-infra-red autofluorescence (NIR-AF) image(s), confocal scanning laser ophthalmoscopy (cSLO) image(s), fundus autofluorescence (FAF) image(s), color fundus photography (CFP) image(s), OCT-angiography image(s), fluorescence life-time imaging ophthalmology (FLIO) image(s), fluorescence lifetime imaging (FLIM) image(s), polarized fluorescence microscopy image(s) and/or others.

In a preferred embodiment, both, first subject data and second subject data (and further subject data if present) comprise at least one OCT image of an eye, or a part thereof, of a subject.

OCT can be used to generate cross-sectional or three-dimensional images (scans) by utilizing low coherence interferometry to detect and measure the depth and magnitude of back scattered light. A two-dimensional, cross-sectional retinal image is produced as the light source scans across the retina, stacking and aligning consecutive axial-scans (A-scans) side by side to produce a two-dimensional transverse-scan (B-scan). Therefore, an A-scan can generally include data from the cornea to the retina, and a B-scan can include cross-sectional data from a medial border to a lateral border of the eye and from the cornea to the retina. The images produced resemble histological sections, with contrast produced by differences in the refractive index and scattering properties of the different retinal layers.

In other words: the term "A-scan" describes the light reflectivity associated with different sample depths; the term "B-scan" refers to the use of cross-sectional views of tissues formed by assembly of a plurality of A-scans. Three-dimensional C-scans (3D macular cube scan) can be formed by combining a plurality of B-scans.

Details about OCT and how to create OCT scans can be found e.g. in textbooks such as Aumann S., Donner S., Fischer J., Müller F.: Optical Coherence Tomography (OCT): Principle and Technical Realization, (2019), in: Bille J. (eds) High Resolution Imaging in Microscopy and Ophthalmology. Springer, Cham. https://doi.org/10.1007/978-3-030-16638-0_3.

The at least one OCT image can be at least one A-scan, at least one B-scan and/or at least one C-scan.

In a preferred embodiment, first subject data and second subject data (and further subject data if present) comprise a at least one OCT B-scan taken from one or both eyes of the subject. Preferably, first subject data and second subject data (and further subject data if present) comprise a plurality of OCT B-scans, the OCT B-scans being parallel to each other and forming planes that are parallel to the direction of the beam of light.

The term "plurality" of OCT B-scans means a number n of OCT B-scans, wherein *n* is an integer equal to or greater than 2, for example, *n* = 2, 3, 4, 5, 6, 7, 8, 9, 10, 11, 12, 13, 14, 15, 16, 17, 18, 19, 20 or any other number.

The spatial distance between each OCT B-scan plane within the plurality of OCT B-scans may vary in the range of 0 to a half of the beam width in micrometers. For example, the spatial distance may vary in the range of 0 to 15 micrometers.

It also possible that the subject data comprise images of an eye, or a part thereof, of the subject generated by different imaging techniques, e.g. one or more OCT scans as well as one or more fluorescein angiography image(s) and/or color fundus photography image(s) and/or other ocular image(s).

Instead of or in addition to one or more images of a subject's eye or a part thereof, one or more metrics e.g. derived from one or more images can be used as subject data. Examples of metrices include: a volume of subretinal fluid in a region of interest, a volume of intraretinal fluid within the region of interest, a total retinal volume within the region of interest, a subretinal fluid index, an intraretinal fluid index, a total fluid index and others. Further examples of metrices that can be used as (addition) input data are provided below.

In addition to the at least one image of an eye, or a part thereof, of the subject, additional subject data can be used. Such additional subject data can be anatomic or physiology data of the subject, such as information about subject's height and weight, gender, age, vital parameters (such as blood pressure, breathing frequency and heart rate), further symptoms, medical history, concomitant medication, any kind of eye-related assessment information such as other diseases, information about wet AMD status, time span since first diagnosis, actual visual acuity, deterioration of actual visual acuity over time, intraocular pressure, etc. Further additional patient data can be found in publications such as: M. Ohji et al.: Efficacy and Safety of Intravitreal Aflibercept Treat-and-Extend Regimens in Exudative Age-Related Macular Degeneration: 52- and 96-Week Findings from ALTAIR, Adv Ther 37, 1173-1187 (2020), https://doi.org/10.1007/s12325-020-01236-x.

Besides subject data (data characterizing the subject), data about the at least one image of a subject's eye, or a part thereof, can also be used for predicting a change related to macular fluid, such as type of image, scanned area(s), magnification, resolution, measurement parameters, wavelength(s) of light used for the creation of the image(s), time the image was created (time stamp) and/or other metadata.

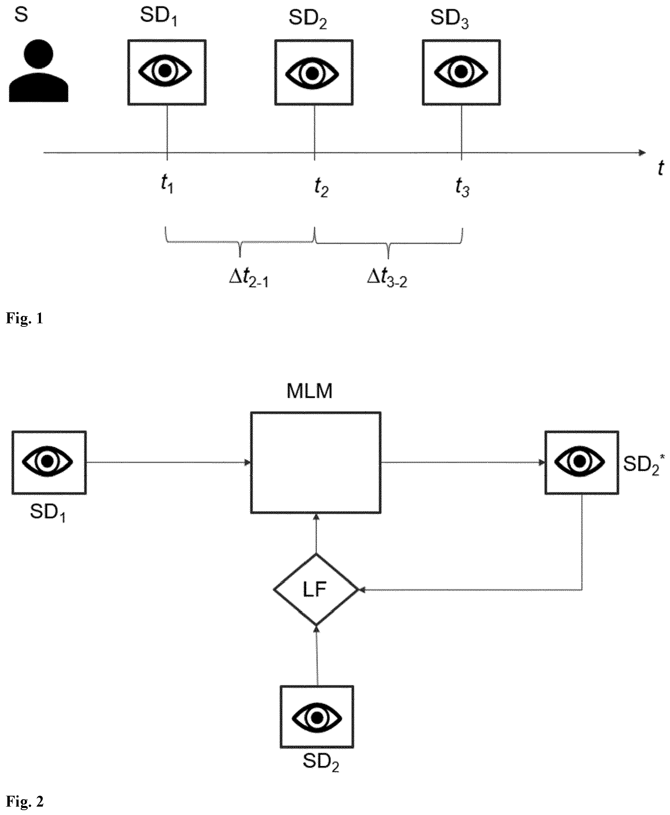

Fig. 1 shows schematically by way of example the collection of subject data for training the machine learning model. Fig. 1 shows a timeline t. At certain points in time, subject data are generated, the subject data characterizing a macular fluid in an eye of a subject S at the respective point in time. At a first point in time *t*₁ first subject data SD₁ are generated/collected/determined, the first subject data SD₁ characterizing a macular fluid in the eye of the subject S at the first point in time *t*₁. At a second point in time *t*₂ second subject data SD₂ are generated/collected/determined, the second subject data SD₂ characterizing the macular fluid in the eye of the subject S at the second point in time *t*₂. At a third point in time *t*₃ third subject data SD₃ are generated/collected/determined, the third subject data SD₃ characterizing the macular fluid in the eye of the subject S at the third point in time *t*₃. The time interval (time period) Δ*t*₂₋₁ between the first point in time *t*₁ and the second point in time *t*₂ can be the same as or can be different from the time interval Δ*t*₃₋₂ between the second point in time *t*₂ and the third point in time *t*₃. For generating the training data set, subject data of a multitude of subjects are generated and/or collected and/or determined.

Fig. 2 shows schematically by way of example the training of the machine learning model. During training, first subject data SD₁ are inputted into the machine learning model MLM. The machine learning model MLM is configured to generate, on the basis of the inputted data, predicted second subject data SD₂*. The predicted subject data SD₂* are compared with real second subject data SD₂. A loss function LF is used to compute a loss value. The loss value quantifies the deviation(s) between the predicted second subject data SD₂* and the real subject data SD₂. During training, parameters of the machine learning model MLM are modified in order to reduce the loss value to a defined minimum.

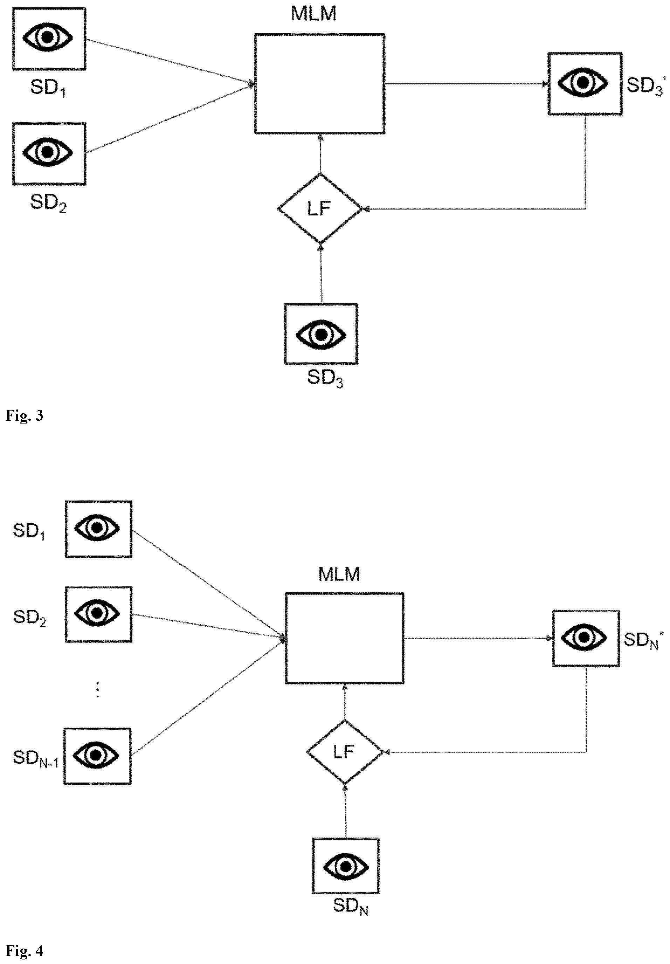

Fig. 3 shows schematically by way of example another variant of the training of the machine learning model. During training, first subject data SD₁ and second subject data SD₂ are inputted into the machine learning model MLM. The machine learning model MLM is configured to generate, on the basis of the inputted data, predicted third subject data SD₃*. The predicted subject data SD₃* are compared with real third subject data SD₃. A loss function LF is used to compute a loss value. The loss value quantifies the deviation(s) between the predicted third subject data SD₃* and the real subject data SD₃. During training, parameters of the machine learning model MLM are modified in order to reduce the loss value to a defined minimum.

Fig. 4 shows schematically by way of example another variant of the training of the machine learning model. Subject data characterizing an eye of a subject at a number (N-1) of time points are inputted into the machine learning model MLM, wherein N is an integer equal to or greater than 3. The subject data comprise e.g. first subject data SD₁ characterizing the eye of the subject at a first point in time, second subject data SD₂ characterizing the eye of the subject at a second point in time, and subject data SD_{N-1} characterizing the eye of the subject at an (N-1)^{th} point in time. Depending on the number N, there may be third, fourth, fifth, ... subject data characterizing the subject's eye at a third, fourth, fifth, ... point in time. The machine learning model MLM is configured to generate, on the basis of the inputted data, predicted subject data SD_{N}*, wherein the predicted subject data SD_{N}* characterized the eye of the subject at an N^{th} point of time. Preferably (but not necessarily), the N^{th} point in time is later than the (N-1)^{th} point in time, and later than the second point in time, and later than the first point in time. The predicted subject data SD_{N}* is compared with real subject data SD_{N}. A loss function LF is used to compute a loss value. The loss value quantifies the deviation(s) between the predicted third subject data SD_{N}* and the real subject data SD_{N}. During training, parameters of the machine learning model MLM are modified in order to reduce the loss value to a defined minimum.

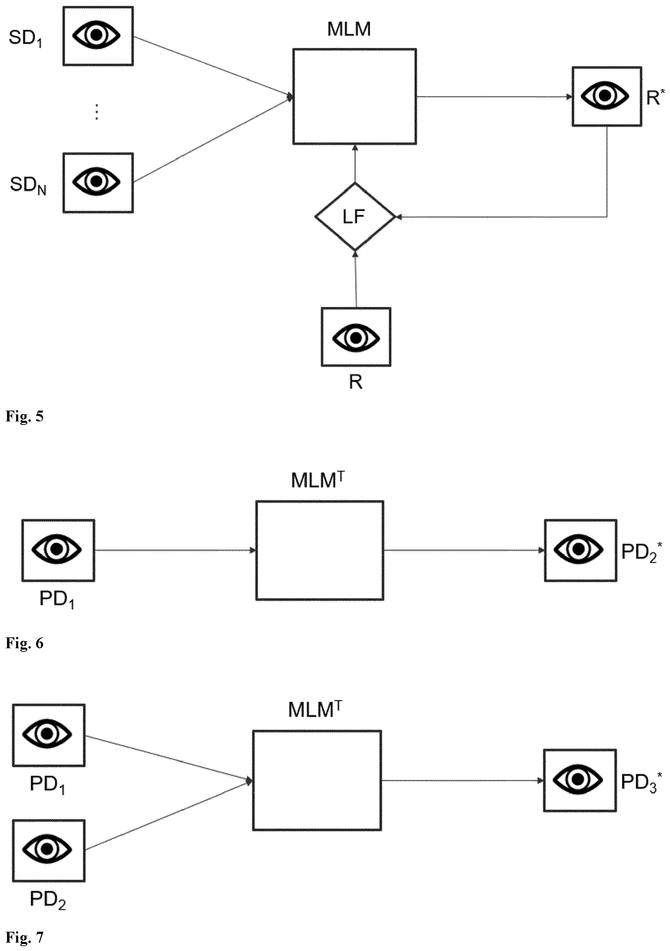

Fig. 5 shows schematically by way of example another variant of the training of the machine learning model. Subject data characterizing an eye of a subject at a number N of time points are inputted into the machine learning model MLM, wherein N is an integer equal to or greater than 2. The machine learning model MLM is configured to generate, on the basis of the inputted data, a prediction result R*. The prediction result relates to a change of macular fluid in the eye of the patient. The prediction result R* may indicate whether the amount of fluid will increase or decrease or stay the same, or the prediction result R* may indicate the magnitude of a change (e.g. a change in volume, size, number, ...). The prediction result R* is compared with a measured result R. A loss function LF is used to compute a loss value. The loss value quantifies the deviation(s) between the predicted result R* and the measured result R. During training, parameters of the machine learning model MLM are modified in order to reduce the loss value to a defined minimum.

The machine learning model according to the present disclosure can be or comprise an artificial neural network.

An artificial neural network (ANN) is a biologically inspired computational model. An ANN usually comprises at least three layers of processing elements: a first layer with input neurons (nodes), a kth layer with at least one output neuron (node), and *k*-2 inner layers, where *k* is an integer greater than 2.

In such a network, the input neurons serve to receive the input data. If the input data constitute or comprise an image, there is usually one input neuron for each pixel/voxel of the input image; there can be additional input neurons for additional input data such as data about the object represented by the input image, measurement conditions, data about the subject and/or the like. The output neurons serve to output the output data.

The processing elements of the layers are interconnected in a predetermined pattern with predetermined connection weights therebetween. Each network node usually represents a (simple) calculation of the weighted sum of inputs from prior nodes and a non-linear output function. The combined calculation of the network nodes relates the inputs to the outputs.

When trained, the connection weights between the processing elements in the ANN contain information regarding the relationship between the input data and the output data which can be used to predict new output data from a new input data.

Each network node represents a calculation of the weighted sum of inputs from prior nodes and a non-linear output function. The combined calculation of the network nodes relates the inputs to the output(s).

Separate networks can be developed for each property measurement or groups of properties can be included in a single network. Preferably, different dimensions of the patient data (and/or optionally additional data) are combined at the end of the algorithm.

Training estimates network weights that allow the network to calculate (an) output value(s) close to the measured output value(s). A supervised training method can be used in which the output data is used to direct the training of the network weights. The network weights can be initialized with small random values or with the weights of a prior partially trained network. The training data inputs are applied to the network and the output values are calculated for each training sample. The network output values are compared to the measured output values. A backpropagation algorithm can be applied to correct the weight values in directions that reduce the error between measured and calculated outputs. The process is iterated until no further reduction in error can be made or until a predefined prediction accuracy has been reached.

A cross-validation method can be employed to split the data into training and validation data sets. The training data set is used in the backpropagation training of the network weights. The validation data set is used to verify that the trained network generalizes to make good predictions. The best network weight set can be taken as the one that best predicts the outputs of the training data. Similarly, varying the number of network hidden nodes and determining the network that performs best with the data sets optimizes the number of hidden nodes.

In a preferred embodiment of the present invention, the machine learning model is or comprises a convolutional neural network (CNN).

A CNN is a class of deep neural networks, most commonly applied to analyzing visual imagery (such as OCT scans and fluorescein angiography images). A CNN comprises an input layer with input neurons, an output layer with at least one output neuron, as well as multiple hidden layers between the input layer and the output layer.

The hidden layers of a CNN typically consist of convolutional layers, ReLU (Rectified Linear Units) layer i.e. activation function, pooling layers, fully connected layers and normalization layers.

The nodes in the CNN input layer are organized into a set of "filters" (feature detectors), and the output of each set of filters is propagated to nodes in successive layers of the network. The computations for a CNN include applying the convolution mathematical operation to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various color components or grey scale values of an input image. The convolution kernel can be a multidimensional array of parameters, where the parameters are adapted by the training process for the neural network.

By analysis of the CNN, one can reveal patterns in the data which are not obvious and were used preferred (i.e., weighted more strongly) by the CNN while analyzing the training data. This explainable AI approach helps to generate trust in the performance of the predictive machine learning model.

The machine learning model may be or comprise a recurrent neural network. Recurrent neural networks (RNNs) are a family of feedforward neural networks that include feedback connections between layers. RNNs enable modeling of sequential data by sharing parameter data across different parts of the neural network. The architecture for an RNN includes cycles. The cycles represent the influence of a present value of a variable on its own value at a future time, as at least a portion of the output data from the RNN is used as feedback for processing subsequent input in a sequence.

Once trained, the machine learning model can be used to predict a change related to macular fluid in an eye of a patient.

The prediction is made on the basis of patient data. The patient data comprise information about a macular fluid in an eye of the patient at a first point in time.

The patient data is inputted into the trained machine learning model. The machine learning model generates output data on the basis of the input data. The output data comprises information about a change related to the macular fluid in the eye of the patient.

The output data and/or the information can be outputted, e.g. displayed on a monitor, printed on a printer and/or stored on a data storage.

The predicted change related to the macular fluid can be a prediction about how a macular fluid will look like at a future point in time. In this case, the trained machine learning model can be configured and trained to predict a temporal change of the macular fluid (e.g. volume, size, shape, composition, spatial distribution, and/or the like).

Patient data can comprise different patient data sets, for example, each data set characterizing the macular fluid at a different point in time and/or each data set consisting of data of different modalities such as one or more images and/or one or more text files and/or one or more tables containing numbers.

In a preferred embodiment, the patient data comprise one or more images of the patient's eye or a part thereof, as described above for the subject in connection with the training of the machine learning model.

Such an image (e.g. an OCT image and/or an FA image and/or an CFP image and/or any other spatial representation of the patient's eye or a part thereof) shows a macular fluid in the eye of the patient at a first point in time, and the machine learning model generates, on the basis of such an image, output data, the output data comprising information about a change related to the macular fluid in the eye of the patient at a later (second) point in time.

The output data can e.g. be an information whether the quantity of macular fluid will increase or decrease. In such a case, the output data may be a binary information.

The output data can e.g. be an expected value of how strong an increase or decrease of the amount of macular fluid will be. In such a case, the output data may be a number, e.g. a percentage increase or decrease.

In a preferred embodiment, the output data is an image, the image showing the status of the macular fluid at a later (second) point in time. The status can be the amount of macular fluid, the distribution of macular fluid (compartments), the type of macular fluid, the appearance of macular fluid, the morphology of macular fluid, and/or the like, at the later point in time.

Fig. 6 shows schematically by way of example the use of a trained machine learning model for predicting a change related to macular fluid in an eye of a patient. Patient data PD₁ characterizing the eye of the patient at a first point in time is inputted into the trained machine learning model MLM^{T}. The trained machine learning model MLM^{T} is configured and trained to output predicted patient data PD₂*. The predicted patient data PD₂* characterize the eye of the patient at a second point in time. Preferably, the second point in time is later than the first point in time. The trained machine learning model MLM^{T} may be the machine learning model as discussed with reference to Fig. 2, 3, or 4.

Fig. 7 shows schematically another example of using a trained machine learning model for predicting a change related to macular fluid in an eye of a patient. Patient data PD₁ characterizing the eye of the patient at a first point in time and patient data PD₂ characterizing the eye of the patient at a second point in time are inputted into the trained machine learning model MLM^{T}. The trained machine learning model MLM^{T} is configured and trained to output predicted patient data PD₃*. The predicted patient data PD₃* characterize the eye of the patient at a third point in time. Preferably, the third point in time is later than the first point in time and later than the second point int time. The trained machine learning model MLM^{T} may be the machine learning model as discussed with reference to Fig. 3 or 4.

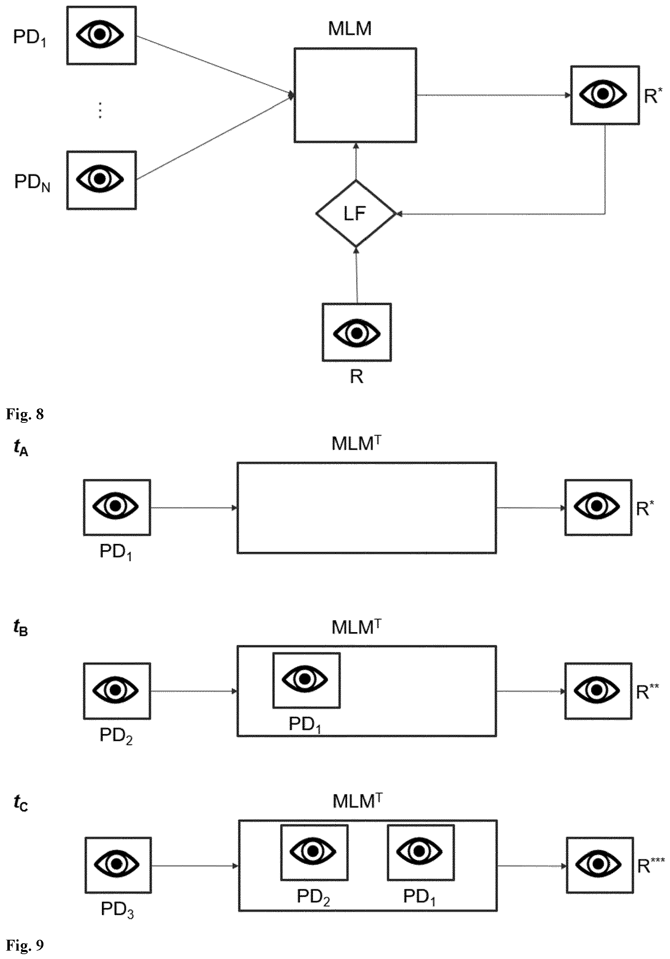

Fig. 8 shows schematically another example of using a trained machine learning model for predicting a change related to macular fluid in an eye of a patient. A number N of patient data sets are inputted into the trained machine learning model MLM^{T}, wherein N is an integer equal to or greater than 2. The number of patient data sets comprises first patient data PD₁ characterizing the eye of the patient at a first point in time, and a N^{th} patient data characterizing the patient's eye at an N^{th} point in time. Depending on the number N, there may be second, third, fourth, fifth, ... patient data characterizing the patient's eye at a second, third, fourth, fifth, ... point in time. The trained machine learning model MLM^{T} is configured and trained to output a prediction result R*. The prediction result R* may indicate whether the amount of fluid will increase or decrease or stay the same, or the prediction result R* may indicate the magnitude of a change (e.g. a change in volume, size, number, ...), or the prediction result R* may comprise a synthetic image (a predicted image) showing the status (amount, distribution, type, appearance, morphology and/or the like) of macular fluid (compartments) at a defined (later) point in time. The trained machine learning model MLM^{T} may be the machine learning model as discussed with reference to Fig. 5.

In a preferred embodiment, the machine learning model is configured and trained to update previous predictions. This is schematically shown in Fig. 9. At a time point *t*_{A}, first patient data PD₁ are available. First patient data PD₁ characterize a patient's eye at a first point in time. The first patient data PD₁ are inputted into a trained machine learning model MLM^{T}. The trained machine learning model MLM^{T} is configured and trained to generate a first prediction result R* on the basis of the first patient data PD₁. The first prediction result R* relates to an expected (predicted) change related to a macular fluid in the eye of the patient. At a time point *t*_{B}, second patient data PD₂ are available. The second patient data PD₂ may characterize a patient's eye at a second point in time and/or the second patient data PD₂ may comprise additional data characterizing the patient's eye at the first point in time. The second patient data PD₂ are inputted into the trained machine learning model MLM^{T}. The trained machine learning model MLM^{T} is configured to recognize that new and/or additional data are available and generates a second (updated) prediction result R**, on the basis of the first patient data PD₁ and the second patient data PD₂. At a time point *t*_{C}, third patient data PD₃ are available. The third patient data PD₃ may characterize a patient's eye at a third point in time and/or the third patient data PD₃ may comprise additional data characterizing the patient's eye at the first point in time and/or at the second point in time. The third patient data PD₃ are inputted into the trained machine learning model MLM^{T}. The trained machine learning model MLM^{T} is configured to recognize that new and/or additional data are available and generates a third (updated) prediction result R***, on the basis of the first patient data PD₁, the second patient data PD₂, and the third patient data PD₃.

The first patient data may comprise an OCT image created by an ophthalmologist. The second patient data and/or third patient data may comprise an OCT image created by the patient himself/herself using a home-based OCT device. Usually, the image quality of home-based OCT devices cannot compete with the quality of the devices used by ophthalmologists. However, the images produced by such home-based OCT devices can be used to update a previous prediction. An advantage of a home-based OCT device is that it allows patients to acquire images in the comfort of their homes (see e.g. J. B. Miller et al.: The Future of Home-Based OCT for Retina Patients, Retina Today, Nov. 2019, pages 35-37). Images generated by the patient himself/herself in connection with the trained machine learning model can provide both, the patient and the ophthalmologist, with current (updated) predictions without the patient having to go to the ophthalmologist's office.

In a preferred embodiment, the machine learning model comprises a segmentation unit.

The term segmentation, as it is used herein, refers to the process of partitioning an image into multiple segments (sets of pixels/voxels, also known as image objects). The goal of segmentation is to simplify and/or change the representation of an image into something that is more meaningful and easier to analyze. Image segmentation is typically used to locate objects and boundaries (lines, curves, etc.) in images. More precisely, image segmentation is the process of assigning a label to every pixel/voxel in an image such that pixels/voxels with the same label share certain characteristics.

The segmentation unit can be configured to receive an image of a patient's eye or a part thereof, to identify macular fluid, and to mark regions in the image showing the identified macular fluid. Marking can be done e.g. by displaying pixels/voxels representing the macular fluid in a pre-defined color and/or putting frames in a pre-defined color around regions in the image which represent the macular fluid.

The segmentation unit can be part of the trained machine learning model or a separate unit.

Methods of identifying macular fluid and segmenting regions containing (different) macular fluid(s) are disclosed in prior art (see e.g. WO2020/234640A1; US2020/0077883A1; C. S. Lee et al.: Deep-learning based, automated segmentation of macular edema in optical coherence tomography, 2017, Biomedical Optics Express 8(7), 3440ff; Y. Guo et al.: Automated Segmentation of Retinal Fluid Volumes From Structural and Angiographic Optical Coherence Tomography Using Deep Learning; Translational Vision Science & Technology October 2020, Vol.9, 54ff; J. Wang et al.: Automated volumetric segmentation of retinal fluid on optical coherence tomography, Biomed Opt Express. 2016 Apr 1, 7(4): 1577-1589; D. Lu et al.: Retinal Fluid Segmentation and Detection in Optical Coherence Tomography Images using Fully Convolutional Neural Network, arXiv:1710.04778 [cs.CV]).

For example, the segmentation unit can be configured to identify in the image pixels/voxels which represent intraretinal fluid, subretinal fluid and/or subretinal pigment epithelium fluid. The segmentation unit can be configured to identify cystic edema in the image. The segmentation unit can be configured to identify regions comprising diffuse intraretinal fluid in the image. The segmentation unit can be configured to determine volumes of macular fluids as well as a total retinal volume. The segmentation unit can be configured to determine the total retinal volume within a region of interest, the volume of subretinal fluid within the region of interest, and/or the volume of intraretinal fluid within the region of interest.

The segmentation unit can be configured to generate one or more metrics indicative of retinal thickness and the prevalence of macular fluid within and underneath the retina within a region of interest, including, for example, the eye as a whole, the macular region, a specific retinal layer or portion of a retinal layer, retinal bands or zones, or another designated region such as a selected subfield in the eye.

Another metric can be the dry retinal volume, defined as the difference between the total retinal volume and a sum of the intraretinal fluid volume and the subretinal fluid volume.

Another metric can be the total fluid index, which is defined as the ratio of the total fluid volume, that is, the sum of the intraretinal fluid volume and the subretinal fluid volume, to the total retinal volume.

Another metric can be the subretinal fluid index which is defined as the ratio of the subretinal fluid volume to the total retinal volume.

Another metric can be the intraretinal fluid index which is defined as the ratio of the intraretinal fluid volume to the total retinal volume.

Another metric can be the pigment epithelial detachment volume (see e.g. https://doi.org/10.3928/23258160-20150610-07).

Another metric can be the central retinal subfield thickness which is an objective measurement of macular thickness readily available on OCT imaging. The central retinal subfield is defined as the circular area 1 mm in diameter centered around the center point of the fovea, with its thickness provided as a quantifiable value on OCT software (see e.g. DOI: 10.1016/j.ophtha.2008.12.033).

Another metric can be the standard deviation of the intraretinal fluid volume and/ or of the subretinal fluid volume and/or of the pigment epithelial detachment volume and/or the central retinal subfield thickness (see e.g. https://doi.org/10.1038/s41433-020-01354-4).

Further metrics which can be obtained from one or more ocular images of a subject and which can be used as biomarkers (and as additional input for the machine learning model) are disclosed in the scientific literature (see e.g. M. Gemayel et al.: Retinal OCT Biomarkers for Clinicians and Clinical Researchers, Retinal Physician, Volume: 18, Issue: April 2021, pages: 28-34).

The segmentation unit can be configured to provide segmented images (images in which one or more macular fluids are identified and in which image objects representing the one or more macular fluids are labeled accordingly) and/or metrics determined from images as input data for training the machine learning model and/or for using the trained machine learning model for making predictions.

In a preferred embodiment of the present disclosure, the segmentation unit is configured to generate, on the basis of one or more images of a patient's eye or a part thereof, one or more segmented images of the patient's eye or a part thereof, wherein one or more regions in the segmented images representing one or more macular fluids in the eye of the patient are labeled.

In a preferred embodiment of the present disclosure, the segmentation unit is configured to provide the one or more segmented images as input data for the trained machine learning model, and the trained machine learning model is configured and trained to generate, on the basis of the one or more segmented images, and optionally on the basis of additional patient data, one or more predicted images, the one or more predicted images showing the patient's eye or a part thereof at a future point in time. Preferably, one or more regions in the predicted images that represent one or more macular fluids are also labeled.

In a preferred embodiment, the one or more segmented images and the predicted images can be displayed together on a monitor.

In a preferred embodiment, the one or more segmented images and the predicted images can be superimposed. A slide control may be provided by a graphical user interface. If the slide control is placed at one side, one or more segmented images may be displayed. If the slide control is placed at the other side, the one or more predicted images are displayed. If the slide control is moved from the one side to the other side, the one or more segmented images may morph into the one or more predicted images.

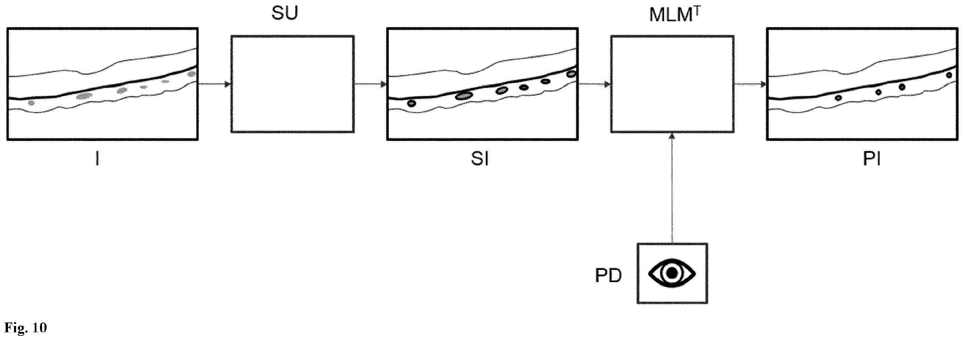

Fig. 10 shows schematically another example of using a trained machine learning model for predicting a change related to macular fluid in an eye of a patient. An image I is inputted into a segmentation unit SU. In the example, the image is an OCT B-scan, however, it can also be another ocular image of the patient. It is also possible that more than one image is inputted into the segmentation unit SU. It is also possible that additional (patient) data are inputted into the segmentation unit SU. The image I shows some fluid compartments in the eye of the patient. The segmentation unit SU is configured to generate a segmented image SI. In the segmented image, regions representing the fluid compartments are framed. The segmented image SI is inputted into the trained machine learning model MLM^{T}. Optionally, additional (patient) data PD can be inputted into the trained machine learning model MLM^{T} as well. The trained machine learning model MLM^{T} is configured and trained to generate a predicted image PI on the basis of the inputted data. The predicted image PI shows the same region as the segmented image, however, at a future point in time. In the example depicted in Fig. 10, the predicted image shows that some fluid compartments will disappear, and the size of the other fluid compartments will decrease. In case of the example depicted in Fig. 10, the predicted change related to macular fluid in the eye of the patient may be the predicted result of a treatment, e.g. after administering an anti-VEGF agent. In the example depicted in Fig. 10, the segmentation unit SU is separate from the trained machine learning model MLM^{T}. As described above, the segmentation unit can also be part of the trained machine learning model MLM^{T}.

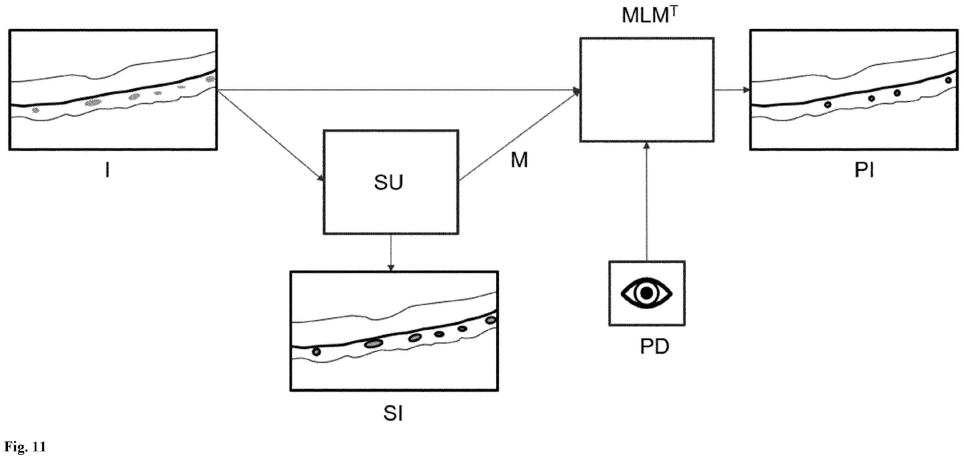

Fig. 11 shows schematically another example of using a trained machine learning model for predicting a change related to macular fluid in an eye of a patient. An image I is inputted into a segmentation unit SU. The image I shows some fluid compartments in the eye of the patient. The segmentation unit SU is configured to generate a segmented image SI. In the segmented image SI, regions representing the fluid compartments are framed. In addition, the segmentation unit SU is configured to determine one or more metrics from the image I, such as the volumes of the fluid compartments. The (original) image I is inputted into a trained machine learning model MLM^{T}. In addition, the one or more metrics are inputted into the trained machine learning model MLM^{T}. Optionally, additional (patient) data PD can be inputted into the trained machine learning model MLM^{T} as well. The trained machine learning model MLM^{T} is configured and trained to generate a predicted image PI on the basis of the inputted data. The predicted image PI shows the same region as the (original) image I, however, at a future point in time.

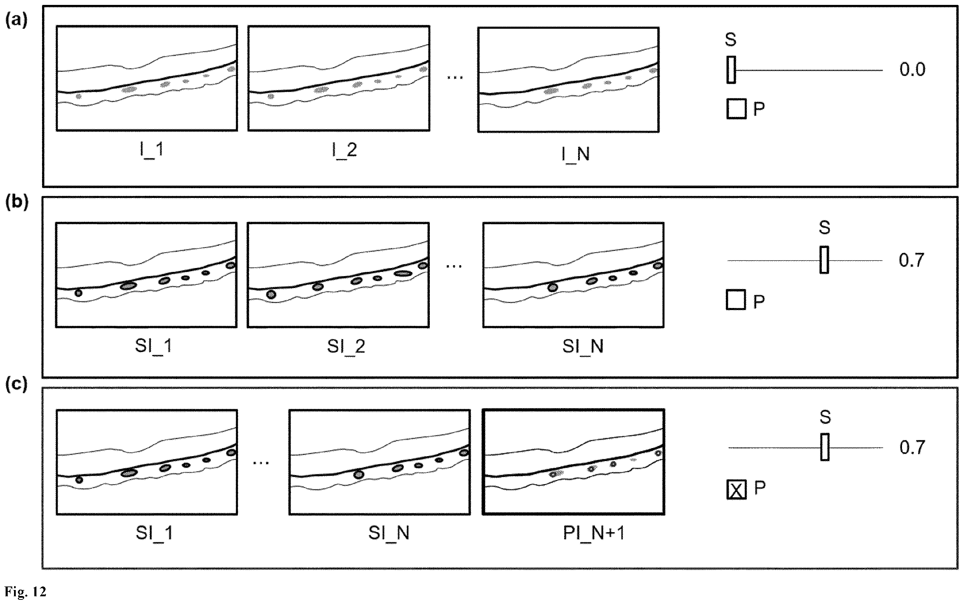

Fig. 12 shows schematically another example of using a trained machine learning model for predicting a change related to macular fluid in an eye of a patient. Fig. 12 (a), Fig. 12 (b), and Fig. 12 (c) show a graphical user interface provided by a computer program on a display of a computer system. The computer program can be used e.g. by an ophthalmologist. The computer program can be used to display (original) images, segmented images, and/or predicted images of an eye of a patient. The graphical user interface provides a slide control S and a tick box P. In Fig. 12 (a), the slide control S is placed at one side (at the left side), and the tick box P is not activated. The effect is that computer program displays the images I_1, I_2, ..., I_N. Each image shows macular fluid in the eye of the patient at a different point in time. Image I_1 shows macular fluid compartments at a first point in time, image I_2 shows macular fluid compartments at a second point in time, and I_N shows macular fluid compartments at an N^{th} second point in time. It is possible that the computer program displays additional images showing macular fluid compartments in the eye of the patient at different additional point in times, as indicated by the three dots (...). In Fig. 12 (b) the slide control S was moved 70% (0.7) to the other side (the right side). This movement has the effect that segmented images SI_1, SI_2, ..., SI_N are displayed superimposed to the original images I_1, 1_2, ..., I_N. In the segmented images SI_1, SI_2, ..., SI_N the macular fluid compartments are labelled with a frame. The degree of superimposition can be adjusted by a user using the slide control. In other words: the user can show and hide the labels. In Fig. 12 (c) the tick box P is activated. The result is that a predicted image PI_N+1 is displayed (in addition to the (original) images and/or the segmented images). The predicted image PI_N+1 shows the expected (predicted) status of the macular fluid compartments in the eye of the patient at an (N+1)^{th} point in time, which is usually a future point in time. The predicted image PI_N+1 is a predicted segmented image (an image showing the predicted segmentation); it shows the predicted segmented macular fluid compartments at the (N+1)^{th} point in time. Since the slide control S is still shifted 70% (0.7) to the right side, the predicted image PI_N+1 is overlaid with an original image. The overlaid original image is preferably the last original image generated (in this example the image I_N), but it is also possible that the user (e.g. the ophthalmologist) can decide which image is used for superimposition.

The operations in accordance with the teachings herein may be performed by at least one computer system specially constructed for the desired purposes or at least one general-purpose computer system specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g. smartphone); all these systems can be utilized for carrying out the invention.

The term "process" as used above is intended to include any type of computation or manipulation or transformation of data represented as physical, e.g. electronic, phenomena which may occur or reside e.g. within registers and/or memories of at least one computer or processor. The term processor includes a single processing unit or a plurality of distributed or remote such units.

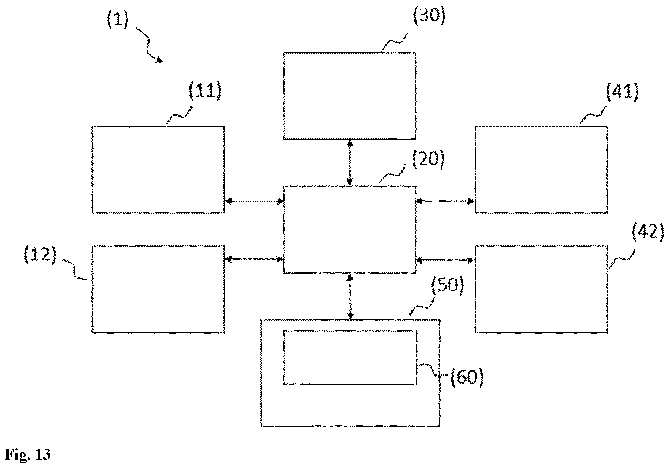

Fig. 13 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

The machine learning model, the trained machine learning model and the segmentation unit may be stored in the memory (50).

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method for predicting a change of a macular fluid in an eye of a patient, the method comprising the steps:
• providing a trained machine learning model,
• receiving patient data, the patient data comprising information about a macular fluid in an eye of a patient,
• inputting the patient data into the trained machine learning model,
• receiving from the trained machine learning model information about a change related to the macular fluid in the eye of the patient,
• outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.

2. The method according to claim 1, wherein the macular fluid is intraretinal fluid, subretinal fluid and/or subretinal pigment epithelium fluid in a region of interest within the eye of the patient.

3. The method according to claim 2, wherein the region of interest is the eye as a whole, the macular region, a specific retinal layer or portion of a retinal layer, retinal bands or zones, or a selected subfield in the eye.

4. The method according to any one of claims 1 to 3, wherein the change related to the macular fluid is a change in nature, texture, composition, shape, quantity, and/or spatial distribution of one or more macular fluids in the eye of the patient over time.

5. The method according to any one of claims 1 to 4, wherein the patient data comprises one of more images of the eye of the patient or a part thereof, preferably one or more OCT images.

6. The method according to any one of claims 1 to 5, wherein the patient data comprise additional patient data, wherein the additional patient data are selected from: anatomic and/or physiology data of the patient, vital parameters, symptoms, medical history, concomitant medication, any kind of eye-related assessment information, information about wet AMD status, time span since first diagnosis, actual visual acuity, deterioration of actual visual acuity over time, intraocular pressure.

7. The method according to any one of claims 1 to 6, wherein the patient data comprise one or more metrics determined from one or more images of the patient's eye or a part thereof.

8. The method according to claim 7, wherein the one or more metrics are selected from: total retinal volume within a region of interest, volume of subretinal fluid within a region of interest, volume of intraretinal fluid within a region of interest, a dry retinal volume within a region of interest, a total fluid index, a subretinal fluid index, an intraretinal fluid index, a pigment epithelial detachment volume in a region of interest, a central retinal subfield thickness, a standard deviation of an intraretinal fluid volume and/ or of a subretinal fluid volume and/or of a pigment epithelial detachment volume and/or of a central retinal subfield thickness.

9. The method according to any one of claims 1 to 8, wherein the information about the change related to the macular fluid in the eye of the patient received from the trained machine learning model is one or more predicted images showing the eye of the patient or a part thereof at a future point in time.

10. The method according to any one of claims 1 to 9, wherein the method further comprises:
• providing a segmentation unit,
• inputting one or more images into the segmentation unit, the one or more images representing the eye of the patient or a part thereof,
• receiving from the segmentation unit one or more segmented images and/or metrics determined from the one or more images,
• inputting the one or more segmented images and/or metrics as patient data into the trained machine learning model,
• receiving from the trained machine learning model information about a change related to the macular fluid in the eye of the patient, wherein the information comprises one or more predicted images showing the eye of the patient or a part thereof at a future point in time.

11. The method according to any one of claims 1 to 10, wherein the change related to the macular fluid is caused by treatment of the eye of the patient with a defined dose of a medicament, preferably of a medicament comprising a VEGF antagonist, more preferably of a medicament containing Aflibercept and/or caused by non-treatment.

12. The method according to any one of claims 1 to 11, wherein the method further comprises the steps:
• receiving from a user information about a future point in time,
• receiving from the trained machine learning information about the status of macular fluid in the eye of the patient at the future point in time,
• outputting the information.

13. The method according to any one of claims 1 to 12, wherein the method comprises the steps:
• receiving patient data, the patient data comprising at least one image of the eye of the patient,
• inputting the at least one image into a segmentation unit,
• receiving from the segmentation unit at least one segmented image, the at least one segmented image showing one or more labelled macular fluid compartments,
• inputting the patient data and/or optionally the at least one segmented image and/or optionally one or more metrics obtained from the segmentation unit into the trained machine learning model,
• receiving from the trained machine learning model a predicted image, the predicted image being a segmented image in which labelled macular fluid compartments are shown at a future point in time,
• outputting the predicted image.

14. A computer system comprising:
a processor; and
a memory storing an application program configured to perform, when executed by the processor, an operation, the operation comprising:
• receiving patient data, the patient data comprising information about a macular fluid in an eye of a patient,
• inputting the patient data into the trained machine learning model,
• receiving from the trained machine learning model information about a change related to the macular fluid in the eye of the patient,
• outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.

15. A non-transitory computer readable medium having stored thereon software instructions that, when executed by a processor of a computer system, cause the computer system to execute the following steps:
• receiving patient data, the patient data comprising information about a macular fluid in an eye of a patient,
• inputting the patient data into the trained machine learning model,
• receiving from the trained machine learning model information about a change related to the macular fluid in the eye of the patient,
• outputting the information,
wherein the machine learning model is trained on the basis of training data to predict a change related to a macular fluid in an eye of a subject on the basis of subject data, wherein the training data comprise, for each subject of a multitude of subjects, i) first subject data comprising information about the macular fluid in an eye of the subject at a first point in time and ii) second subject data comprising information about the macular fluid in the eye of the subject at a second point in time.
